# EUROPEAN PATENT APPLICATION

(11) **EP 3 070 177 A1**
(43) Date of publication of application: **21.09.2016**
(21) Application number: 14861672.5
(22) Date of filing: 14.11.2014
(51) Int. Cl.: C12Q 1/68, G01N 33/68, C12N 15/09

(54) **METHOD FOR PREDICTING LONG-TERM EFFICACY OF VEGF INHIBITOR**

(30) Priority: 15.11.2013 JP 2013237162
(71) Applicant: Toppan Printing Co., Ltd., Tokyo 110-0016 (JP)
(72) Inventor: KITANO Shiro, Tokyo 110-0016 (JP); YAMADA Takeshi, Tokyo 113-8603 (JP)
(74) Representative: TBK
(86) International application number: PCT/JP2014/080246
(87) International publication number: WO 2015/072555

(57) **Abstract**

A method for predicting long-term efficacy of a VEGF inhibitor of the present invention is a method for predicting long-term efficacy of a VEGF inhibitor exerted on a tumor of a subject, the method includes determining whether or not an RAS gene-derived nucleic acid or an RAS protein is present in a blood sample collected from a subject, determines whether the RAS gene-derived nucleic acid in the blood sample is a wild type or a mutant or whether the RAS protein in the blood sample is a wild type or a mutant, determining that an antitumor effect resulting from the VEGF inhibitor is highly likely to last for a long period of time in the subject in a case where a wild-type RAS gene-derived nucleic acid or a wild-type RAS protein is detected in the blood sample and a mutant RAS gene-derived nucleic acid or a mutant RAS protein is not detected in the blood sample, determining that the antitumor effect resulting from the VEGF inhibitor is unlikely to last for a long period of time in the subject in a case where the mutant RAS gene-derived nucleic acid or the mutant RAS protein is detected in the blood sample.

SEQUENCE LISTING
<110>  TOPPAN Printing Co., Ltd.
<120>  Method for prediction of long-term response to VEGF inhibitor drug
<130>  PC-19261
<150>  JP2013-237162
<151>  2013-11-15
<160>  6
<170>  PatentIn version 3.1

<210>  1
<211>  188
<212>  PRT
<213>  Homo sapiens
<220>
<223>  KRAS
<400>  1

<210>  2
<211>  3360
<212>  DNA
<213>  Homo sapiens
<400>  2
tcgcttgagg ccaggagttg gagaccagcg tactcaacat agtgagacct tgttataaag      60
aaaaaaaaaa tccaggatta aaaaaaatct ttgatttgtt tgggatttat taatatttac     120
cgtattggaa attaaaacaa ttttttaaaa tgtattcatt taaaaataat aagcccatta     180
cttggtaaca tgaataaaat attttatgaa aaataactat tttccaaaac aaaaccaaaa     240
cttagaaaag tggtattgtt tcacacttca gtaaatctct ttaatgatgt ggcttaatag     300
aagatatgga ttcttatatc tgcatctgca ttcaatctat tatgatcaca catctggaaa     360
acttgtgaaa gaatgggagt taaaagggta aaggacatct taatgttatt atgaaaacag     420
ttttgacctc ttgcacacca gaaaagtctt agtaacctga ggggttccta gaccacattt     480
tgagaactgt tttaggctat gcaaactggt tggggggagg ttggggtagg cagagagcta     540
gaagatacat tttagtgtaa ttctcctcat ctattcctaa ttgctttggc ctacatttga     600
aataaagcgt ggaggcaaac gggataagat acatgtttgt agtggttgtt aacttcaccc     660
tagacaagca gccaataagt ctaggtagag cagagtaagg cggggaacta tgccgtgacc     720
gtgtgtgata caatttttct agcctgtggt gctttttgcg gcagggctta ggagtaaggt     780
tagtatgtta tcatttggga aaccaaatta ttattttggg tcttcagtca attatgatgc     840
tgtgtatatt tagtgtttat ctacaatata tgcacattca ttaatttgga gctactcatc     900
ctataataaa tagttgtgca tttactccca tttttttctg catttctctc cttatttata     960

attatgtgtt acatgaggga aaggaggtga aattaaacat tcatattatt tcaaaaaatt 1020
tgaaacaact aactaaaaaa tatgttttat tttctgtatg gtgtttgtta tacaatctgt 1080
caatattcat gcacctcttg ggagacagtg tatgaaaagc aaagagtaac agtcacatgg 1140
attactgatt actgagatat attcacttgc atcttttttt ttttttgaga cggagtggct 1200
ctgtcgccca ggctggagtg cagtggcgtg atctcggctc actgcaagct ccgcctcctg 1260
ggttcacgcc attcttctgc ctcagcctcc caagtagctg ggactacagg cgcccgccac 1320
cacgcccggc taattttttt atatttttag tagagacggg gtttcaccgg gttagccagg 1380
atggtcttga tctcctgacc tcgtgatcca ccctcctcgg cctcccaaag tgctaggatt 1440
ataggcgtga gccaccgtgc ccggctcact tgcatctctt aacagctgtt ttcttactaa 1500
aaacagtgtt tatctctaat cttttgtttt gtttgtttgt tttgagatgg agtcttactc 1560
cgtcacccaa tctggagtgc agtggcgtga tctgggctca ctgcaacctc tgcctcccgg 1620
gttcaagtga ttctccttcc tcagcctccc cagtagctag gactacagga gagcgccacc 1680
acgcctgatt aatttttgta tttttagtag agagagggtt tcaccatatt ggccaggctg 1740
gtcttgaact cctggcctca ggtgatccac ccgccttggc ctctgaaagt gctgggatta 1800
caggcatgag ccgccgcacc cggctttcta atctttatct ttttttgtgc agcggtgata 1860
caggattatg tattgtactg aacagttaat tcggagttct cttggttttt agctttattt 1920
tccccagaga tttttttttt tttttttttt tttgagacgg agtcttgctc tatcgccagg 1980
ctggagtgca gtggcgccat ctcggctcat tgcaacctcg gactcctatt ttccccagag 2040
atatttcaca cattaaaatg tcgtcaaata ttgttcttct ttgcctcagt gtttaaattt 2100
ttatttcccc atgacacaat ccagctttat ttgacactca ttctctcaac tctcatctga 2160
ttcttactgt taatatttat ccaagagaac tactgccatg atgctttaaa agtttttctg 2220
tagctgttgc atattgactt ctaacactta gaggtggggg tccactagga aaactgtaac 2280
aataagagtg gagatagctg tcagcaactt ttgtgagggt gtgctacagg gtgtagagca 2340
ctgtgaagtc tctacatgag tgaagtcatg atatgatcct ttgagagcct ttagccgccg 2400
cagaacagca gtctggctat ttagatagaa caacttgatt ttaagataaa agaactgtct 2460
atgtagcatt tatgcatttt tcttaagcgt cgatggagga gtttgtaaat gaagtacagt 2520
tcattacgat acacgtctgc agtcaactgg aattttcatg attgaatttt gtaaggtatt 2580
ttgaaataat ttttcatata aaggtgagtt tgtattaaaa ggtactggtg gagtatttga 2640
tagtgtatta accttatgtg tgacatgttc taatatagtc acattttcat tatttttatt 2700
ataaggcctg ctgaaaatga ctgaatataa acttgtggta gttggagctg gtggcgtagg 2760
caagagtgcc ttgacgatac agctaattca gaatcatttt gtggacgaat atgatccaac 2820
aatagaggta aatcttgttt taatatgcat attactggtg caggaccatt ctttgataca 2880
gataaaggtt tctctgacca ttttcatgag tacttattac aagataatta tgctgaaagt 2940
taagttatct gaaatgtacc ttgggtttca agttatatgt aaccattaat atgggaactt 3000
tactttcctt gggagtatgt cagggtccat gatgttcact ctctgtgcat tttgattgga 3060
agtgtatttc agagtttcgt gagagggtag aaatttgtat cctatctgga cctaaaagac 3120
aatcttttta ttgtaacttt tatttttatg ggtttcttgg tattgtgaca tcatatgtaa 3180
aggttagatt taattgtact agtgaaatat aattgtttga tggttgattt ttttaaactt 3240
catcagcagt attttcctat cttcttctca acattagaga acctacaact accggataaa 3300
ttttacaaaa tgaattattt gcctaaggtg tggtttatat aaaggtacta ttaccaactt 3360

<210> 3
<211> 3480
<212> DNA
<213> Homo sapiens
<400> 3
ttttaataga gatggggttt caccatgttg gccaggatgt tcttgatctc ctgacctcat 60
gatccgccca cctcggcctc ccaaagtgtt gggattgcaa gtgtgagcca ccgcgcctag 120
accatggtag ttaattttaa gtgttcaatt cagtgacctt aagtgtgttc ataatgttgt 180
gcaaccatca ccatgttgtc taaccattag cactatctgt tttgagaact tttttttatc 240
atcccaaatt agaattctgt acctgtcaaa tagtccccag taatcctccc tcccccagcc 300
cctggtaatc tgtagtctac ttttcgtctt tttgaatttg cctattttag gttcctcata 360
taagtggaat tatgtggtat ttgtcctttt gtgttggctt acttcattta gcataatgtt 420
ttcaaggttc atctgtgttg tagcatgtat atacaggttg aagcatccgt tatccaaaat 480
ggttgtgacc agaagtggtt tggatttcag attttttttt tggattttgg aatattcata 540
gatacttaac tggttcagca tccctcgtcc aaaaatccaa aatcagatgg agctcagtgg 600
ctcatgcttg taatcccaac acgttgggtg gccaaggcag gaggatcgct tgagcccagg 660
agttcaacca gcctgagcaa cacaagaccc tatctctcca aaaaaaaaaa aaaaaaaaaa 720
aagatgaaag aaaaaaaaat ccaaaatcaa atgctccagt gagcatttcc ttttagcatc 780
atgtcaggct ctaaaagtta caggttttgg agcattttgg atttcagatt tttggattaa 840
cctgcattaa tgctcaacct atatgaaatt ttattccttt ttatggctga ataatgttcc 900
actgtatgta tatactacat tttgtttatc cattcatctg ttaacagaca cttaagttat 960

ttccacattt tgggtattat aaatagtgct gctgcgaaca ttggtgtaca tgtatctgtt 1020
tgagtccctg tttttagtta ttttggttat atacctagga atggaattgc tgatcatatg 1080
gtaattctgt gtttaacttt ttgaggaact accactgttt tccacaatgg catcaccatt 1140
ttacattccc accagcaatg cacaaagatt tcagtgtctg tatccttgct aacacttatt 1200
ttccattttt tgagtttttt tgttttgttt ttttaataat agccaatcct aatgggtatg 1260
tggtagcatc tcatggtttt gattttattt tcctgactat tgatgatgtt gagcatcttt 1320
tcaggtgctt agtggccatt tgtccgtcat ctttggagca ggaacaatgt cttttcaagt 1380
cctttgccca tttttaaatt gaattttttg ttgttgagtt gtatataaca ccttttttga 1440
agtaaaaggt gcactgtaat aatccagact gtgtttctcc cttctcagga ttcctacagg 1500
aagcaagtag taattgatgg agaaacctgt ctcttggata ttctcgacac agcaggtcaa 1560
gaggagtaca gtgcaatgag ggaccagtac atgaggactg ggagggcttt tctttgtgta 1620
tttgccataa ataatactaa atcatttgaa gatattcacc attataggtg ggtttaaatt 1680
gaatataata agctgacatt aaggagtaat tatagttttt attttttgag tctttgctaa 1740
tgccatgcat ataatattta ataaaaattt ttaaataatg tttatgaggt aggtaatatc 1800
cctgttttat aaatgaagtt cttgggggat tagagcagtg gagtaacttg ctccagactg 1860
catcggtagt ggtggtgctg ggattgaaac ctaggcctgt ttgactccac agccttctgt 1920
actcttgact attctacaaa agcaagactt taaacttttt agatacatca ttaaaaaaga 1980
aaaccataaa aaagaatatg aaaagatgat ttgagatggt gtcactttaa cagtcttaaa 2040
agcaatcgtg tgtatagcat agaattgctt ggattggata aacagtggca ttatatattt 2100
taaaaaataa aagttttgaa agattgaaga atttgggcat tacagttctc ttaaatctga 2160
caaagctgca taaaactatt aaaataatca ttattatact attttatatt ctatttcttt 2220
gagggtttag ttttccaaaa actacatatt aagcaaatga atcactcagt ggctatgtca 2280
tataataacg agttagccta gttataagaa gtttaacatt ttatttaaga acattgttac 2340
agcatgttta ctgtatagtc tagtaataga ggaaaagaca tttgggtggg tggtagtggt 2400
agtattttta tagaggagtt accaaatttc agctctatta tccaagttta cccagctaat 2460
ggtgttcgga accgggaatt tgagccaatt ctgactctgt tgtctgctct gctccttctt 2520
ttgtgctgtg tctttgaaag tcacctaaaa ttgtgaggga atgtaatttc accccaaatt 2580
tagagtttat gcacttgtta tattgaaaat gattaacatg tagaagggct tttaatggaa 2640
taagtggtgt agtaacttca gtgttgccta cctagaaatc aaaatctttc tagttgtcca 2700
ctttgttttt tgaaaaagta atatgaaaat tatgttaatg ctttaattca ggtttttgta 2760
aaatattttt tatctttaca catttaacat acgtttctaa aattatagtc tgttatatag 2820
cactttgggt ctagaatttt tcagtagttt ctgttttact attatgatct acctgcatat 2880
taacctatta ggttatagtt ttactatact tctaggtatt tgatcttttg agagagatac 2940
aaggtttctg tttaaaaagg taaagaaaca aaataactag tagaagaagg aaggaaaatt 3000
tggtgtagtg gaaactagga attacattgt tttctttcag ccaaatttta tgacaaaagt 3060
tgtggacagg ttttgaaaga tatttgtgtt actaatgact gtgctataac ttttttttct 3120
ttcccagaga acaaattaaa agagttaagg actctgaaga tgtacctatg gtcctagtag 3180
gaaataaatg tgatttgcct tctagaacag tagacacaaa acaggctcag gacttagcaa 3240
gaagttatgg aattcctttt attgaaacat cagcaaagac aagacaggta agtaacactg 3300
aaataaatac agatctgttt tctgcaaaat cataactgtt atgtcattta atatatcagt 3360
ttttctctca attatgctat actaggaaat aaaacaatat ttagtaaatg tttttgtctc 3420
ttgagagggc attgcttctt aatccagtgt ccatggtact gcttttggct ttggtttctt 3480

<210> 4
<211> 3540
<212> DNA
<213> Homo sapiens
<400> 4
tctacattga aaatttctct tcaattctga gcacatgtta acatttagaa ttcaagaggt 60
ggggattttt ttttcccatg gttacatata tatatatata tatatatata tatatatata 120
tatatatata tataaagaac agggcaacaa atttttgcgt tttctatttc ggtagtactt 180
ttaaaccatt atgtcatgtt tctaggttaa acgttgttgt atttgaagaa ttttactttg 240
gcagaatttt tttgaggatg tgtttatttc tggagaaagg tctcattaaa gaaagacaat 300
acccagaaag ccaacagaaa ttctgttact catttaatgc atttttctga caaaaattat 360
tgccagagag aacctgaatt ttgtttcaaa aatcatcttt gttttaaaaa tgactttttc 420
ttcaggtaaa ataaaataat ttcagttgct attatttaac ctgtttgtat gaagagttta 480
acatatagga aatgaataca taaagatagg aaggaattaa ttgttatatg tagtcatatg 540
tctcttaatg acagggatac tttctaagaa atacattgtt aggtgatttt gtcattgtgc 600
aaacatcata gaatatactt acacaaacct tggtagtata acctactata cacctgggat 660
atgtagtata gtctcttgcc ccagggatac aaacctgtac agtatgtaac tgtactaatg 720
actataaggc aattgttaac acaatggtaa gttttgtgtg tctaaaccta cacttgggct 780
accctaagtt tatatatttt tttaaatttc tgttcaataa taaattaacc ttactttact 840

gtaacttttt aaacttttta atttttccta acattttgac ttttgtaata cagcttaaaa 900
cacacattat acagctatac aaatttttct ttccttatat ctttattctg taagcttttt 960
tccatattta aaattttttg tttgttttta cttattaaac ttttttgtta aaaactaaga 1020
catgcatgca cattaaccta ggcctacaca gggtcaggac catcaatatc attgtcttcc 1080
acttccacat cttgtcccac tggaagatct tcaggggcag taacacacgt ggagctgtca 1140
tctcctataa taacattgcc ttcttttgga atacctcctg aaggacctat ccaaggctgt 1200
ttatagttaa cttttttttt tttttttttt tttttttagt aaataggagg agtacactat 1260
aaaataacaa tataggtgct ataccattat acaactgaca gtgcagtagg tttgtttaca 1320
ccagcatcac cacaaacacg tgagcaatgt gtcgtactac agtgttagga tggctataac 1380
atcactaagc aataggaact tttaaactcc attataatct tatgggacca ctatcacata 1440
tgcaatctcc tgtggaccaa aatgtcatta tgtggtacat gactgtacta agaaattgat 1500
ccatctatat tccatcaatt tgtttagggc tttttctggt tacatttacc tgtgagccca 1560
gaaaaccagt tttgtagaaa ttaacttctg taatgctagg agttaaaaaa aattgctgaa 1620
caacttttac attgttaaac atttaaaaac aagcgttcta gaagtttatc aaatttcata 1680
aaggtgcaaa aatgtaaatg taaatcatta tccagctaat atatatgttg tatttcccta 1740
gtaggagagc atatgtacct cttcctagtt atacaaattt gatatatagt aaagaaacag 1800
taaattctac ttcaagtcat tttgggagga ttaaaaactg aatttctcta gtttgaccat 1860
tgtacagatt tatctggcaa ttttactaaa acctgattta taggttaaac ttggtgtata 1920
tcatatatca ctttacttta gaggaattaa gatttcacat aaatccattt ccaggttcca 1980
aagaccagga agaggcttgg tttttgtttt tctttttact gtctttacag tctccttgac 2040
ttttcttagg agagaaggta ctgagaaaac atgattctaa tatttattat tttttcttcc 2100
aacattttct tatgaaacat tttcaaatac aaaattgagt tttatttaaa acatttgcaa 2160
atatactacc tagattctac cattgttgtt ttatatttgc tttacttaca acttttaaaa 2220
gatgcttttt ataccactga acattttagc ttacatttca caaagaaaag aaaaaattta 2280
agagactttg cataatgttt taaggggttg cagtaaagaa gtgcttctta tattttctta 2340
tgcatacaaa tcagctgggc ttattaaaat ccagattcta attcagaagg tttaggtggg 2400
gaccgagtct gcatttctaa caaactccta ggtggtattt ttcttggtac ttggaccata 2460
ctttgagtag aaaagcagta gaggacataa aaagagtctt gttagtccca ctttgttgct 2520
gtccacttct catttgataa tatcctaaaa tagctgtgtc tcctttttgg tggttgtatg 2580
attactacct cagaagtact aattgattct tgctatttga ccttaatact ttaatataac 2640
acagcattca tatttgatca gaaaactatc tggcttcctt ttataagaga tttttaggtt 2700
ttatacagtt ttgtggcctt gggttttttt gtttgatttg tttttttgaa ggtatataat 2760
atgtaagtag ataaacaaat ttgatttgta gacattttta tgtggatcat ctaattaaaa 2820
atggagggat acagtatgaa agaatacttg tacttcttaa cagagcactc aacctttctt 2880
ttacatcctg tttcactgat gttattatgt aatttatgtt gctaaactat aaattagata 2940
tttaatttct gttctttgat ttccttttat tattaaatgg acttgttgat ttgcctagaa 3000
attaatttgc ctttcaaaag tcttattaat cttcctccgt tgaaattaat ttgatatttg 3060
catgcttctg gaagacttta aagagctatt ccgagtaact gtagagatta taaaatgaaa 3120
tatgggaatt ttaataaatt ttacatctcc agttactggt gaaaatgtca agtcctcctt 3180
tctgcagagt attttgttac tcatctgtta ttcagcttat ttatttattt atttatttat 3240
ttatttttct ttctttcttg tttttttttt ttgagacgga gtcttgcttt gtcgcccagg 3300
ctggagtaca gtggtgggat cttggctcac tgcaggctcc gcctcccggg ttcacaccat 3360
tcttctgcct cagcctccca agtagctggg actacaggca cccgccacca tgccttgcta 3420
aatttttgta tttttagtag agacgggttt cactgtgtta gccaggatgg tctcgatctc 3480
ttgacctcgt gatccacctg cctcggcctc ccaaagtgct gggattacag gcatgagcca 3540

<210> 5
<211> 20
<212> DNA
<213> Artificial Sequence
<220>
<223> Description of Artificial Sequence: KRAS forward primer
<400> 5
gaatggtcct gcaccagtaa 20

<210> 6
<211> 24
<212> DNA
<213> Artificial Sequence
<220>
<223> Description of Artificial Sequence: KRAS reverse primer
<400> 6
gtgtgacatg ttctaatata gtca 24

## Description

### TECHNICAL FIELD

The present invention relates to a method for predicting long-term efficacy of a VEGF inhibitor in a subject by investigating the genotype of an RAS protein in a biological sample collected from the subject in a minimally invasive manner.

Priority is claimed on Japanese Patent Application No. 2013-237162, filed November 15, 2013, the content of which is incorporated herein by reference.

### BACKGROUND ART

Vascular endothelial growth factors (VEGFs) are a group of glycoproteins involved in vasculogenesis (formation of new blood vessels in an avascular portion during a period of embryogenesis) and angiogenesis (formation of blood vessels by branching or elongation of existing blood vessels). VEGF is also called a vascular endothelial cell growth factor, a vascular endothelium growth factor, an angioendothelial growth factor, or the like in some cases. VEGF binds as a ligand to a vascular endothelial growth factor receptor (VEGFR) that is mainly on the surface of vascular endothelial cells, and functions to stimulate the cell division, cell migration, or cell differentiation or to enhance vascular permeability of microvessels. VEGF is also involved in the activation of monocytes·microphages. Furthermore, VEGF is involved not only in angiogenesis in the normal body but also in the process of malignization such as tumor angiogenesis or metastasis of a tumor.

As growth factors involved in vasculogenesis, angiogenesis, and lymphangiogenesis, there are seven growth factors consisting of VEGF-A, VEGF-B, VEGF-C, VEGF-D, VEGF-E, placental growth factor (PIGF)-1, and PIGF-2. These are collectively called the "VEGF family". A growth factor simply called VEGF refers to VEGF-A in some cases. Furthermore, due to alternative splicing, some of the VEGF family members have several subtypes. For example, human VEGF-A generally has four subtypes consisting of 121 amino acids (VEGF-A121), 165 amino acids (VEGF-A165), 189 amino acids (VEGF-A189), and 206 amino acids (VEGF-A206) (NPL 1). Furthermore, rare subtypes such as VEGF-A145 and VEGF-A183 have been reported (NPL 2).

VEGF is a key factor in promoting angiogenesis, and a VEGF signal transmission system is drawing attention as a molecular target for controlling angiogenesis. The expression of VEGF is induced in various cells including cancer cells by a transcription factor, hypoxia-inducible factor 1 (HIF-1), mainly under hypoxic conditions. As a result of mRNA splicing occurring in different ways, VEGF is produced and secreted as homodimers having different sizes. Thereafter, through a VEGF 2 receptor which is mainly receptor-type tyrosine kinase, VEGF promotes the migration·growth of vascular endothelial cells and further enhances the vascular permeability as well.

Hitherto, several research groups have reported markers for predicting the efficacy of VEGF, but markers which will become definite candidates have not yet been reported. Ince WL et al reported that in a phase 3 clinical trial, if an anti-VEGF monoclonal antibody, bevacizumab, is added to the combined modality therapy (IFL regimen) using LV/5-FU + CPT-11 in a first line treatment for patients with metastatic colon·rectal cancer, the value of median OS is extended (comparison test between an IFL + bevacizumab group and an IFL/placebo group, see NPL 3). Furthermore, in order to assess whether the mutation state of KRAS, b-RAF, and p53 or the overexpression of p53 in the nucleus can be a biomarker for predicting the therapeutic effect of bevacizumab, Ince WL et al conducted retrospective analysis for the participants of the aforementioned test.

Johansen et al investigated the connection between the mutation of cancer-related genes and the sensitivity or long-term efficacy of a VEGF inhibitor, and reported that the mutation of the cancer-related genes in tumor tissue functions as a marker for predicting the sensitivity or long-term efficacy of the VEGF inhibitor in gastrointestinal cancer (PTL 1).

### PRIOR ART DOCUMENTS

### PATENT DOCUMENTS

[PTL 1] Published Japanese Translation No. 2012-502285 of the PCT International Publication

### Non-Patent Literature

[NPL 1] Tischer E, et al., Journal of Biological Chemistry, vol. 266, 1991, p.11947-11954.
[NPL 2] Neufeid G, et al., FASEB Journal, vol. 13, 1999, p.9-22.
[NPL 3] Ince WL, et al., Journal of the National Cancer Institute, vol. 97(13), 2005, p.981-989.

### DISCLOSURE OF INVENTION

### PROBLEMS TO BE SOLVED BY THE INVENTION

An object of the present invention is to provide a method for predicting the efficacy of a VEGF inhibitor, which functions as an angiogenesis inhibitor in a tumor (cancer) patient, by using the status of RAS in peripheral blood of the patient as an indicator regardless of the status of RAS in the tumor tissue of the patient.

### MEANS FOR SOLVING THE PROBLEMS

In order to achieve the aforementioned object, the inventors of the present invention conducted intensive research. As a result, they found that when a VEGF inhibitor is administered to a cancer patient, in a case where the status of RAS in tumor tissue is a mutant while the status of RAS in peripheral blood is wild type, the VEGF inhibitor exerts a marked long-term efficacy. They also found that even if mutant RAS is not detected in a patient's tumor tissue having VEGF, if mutant RAS is detected in the patient's peripheral blood, the efficacy of the VEGF inhibitor is low in the patient. Based on the above findings, the inventors accomplished the present invention.

A method for predicting long-term efficacy of a VEGF inhibitor according to an embodiment of the present invention is a method for predicting long-term efficacy of a VEGF inhibitor exerted on a tumor of a subject. The method includes determining whether or not an RAS gene-derived nucleic acid or an RAS protein is present in a blood sample collected from the subject, and determining whether the RAS gene-derived nucleic acid in the blood sample is a wild type or a mutant or whether the RAS protein in the blood sample is a wild type or a mutant, determining that an antitumor effect resulting from the VEGF inhibitor is highly likely to last for a long period of time in the subject in a case where a wild-type RAS gene-derived nucleic acid or a wild-type RAS protein is detected in the blood sample, and a mutant RAS gene-derived nucleic acid or a mutant RAS protein is not detected in the blood sample, and determining that the antitumor effect resulting from the VEGF inhibitor is unlikely to last for a long period of time in the subject in a case where the mutant RAS gene-derived nucleic acid or the mutant RAS protein is detected in the blood sample.

In the aforementioned embodiment, the subject may have undergone surgical resection of a tumor portion or may have been administered with a VEGF inhibitor in the past.

In the aforementioned embodiment, the RAS may be KRas, HRas, or NRAS.

In the aforementioned embodiment, the subject may have been administered with a drug other than the VEGF inhibitor.

In the aforementioned embodiment, the subject may be a tumor patient who has been administered with a VEGF inhibitor and then received antitumor therapy different from the administration of the VEGF inhibitor, and the blood sample may be collected before the tumor patient is going to be administered again with the VEGF inhibitor.

In the aforementioned embodiment, the antitumor therapy may be medication therapy using a chemotherapy agent.

In the aforementioned embodiment, the chemotherapy agent may be one or more agents selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecine, topotecan, teniposide, colcemid, colchicine, paclitaxel, vinblastine, vincristine, and tamoxifen.

In the aforementioned embodiment, the antitumor therapy may be radiotherapy.

In the aforementioned embodiment, the antitumor therapy may be medication therapy using a molecular-targeted drug different type from the VEGF inhibitor which has already been administered to the subject.

In the aforementioned embodiment, the molecular-targeted drug may be one or more drugs selected from the group consisting of cetuximab, panitumumab, bevacizumab, gefitinib, erlotinib, regorafenib, crizotinib, sunitinib, sorafenib, everolimus, trastuzumab, lapatinib, and rituximab.

In the aforementioned embodiment, the antitumor therapy may be combined therapy composed of medication therapy using the molecular-targeted drug and medication therapy using the chemotherapy agent.

In the aforementioned embodiment, the tumor may be a recurrent tumor.

In the aforementioned embodiment, the tumor may be a metastatic focus.

In the aforementioned embodiment, the tumor may be a primary focus.

In the aforementioned embodiment, the tumor may be one or more tumors selected from the group consisting of colorectal cancer, colon cancer, rectal cancer, lung cancer, liver cancer, breast cancer, ovarian cancer, prostatic cancer, renal cancer, esophageal cancer, head and neck cancer, uterine cancer, and cervical cancer.

In the aforementioned embodiment, the tumor may be present in a plurality of places in the body of the subject.

In the aforementioned embodiment, the mutant may be one or more mutations selected from the group consisting of G12A, G12C, G12D, G12R, G12S, G12V, G13D, G12S2, G13A, G13S, G13V, G13R, G13C, Q61H, Q61L, Q61R, A146T, and A146V.

In the aforementioned embodiment, by investigating whether or not the wild-type RAS gene-derived nucleic acid is detected from circulating DNA in the blood sample and investigating whether or not the mutant RAS gene-derived nucleic acid is detected from circulating DNA in the blood sample, whether the RAS gene-derived nucleic acid is present in the blood sample and whether the RAS gene-derived nucleic acid is a wild type or a mutant may be determined.

In the aforementioned embodiment, the blood sample may be peripheral blood, serum, or plasma.

### EFFECTS OF THE INVENTION

According to the aforementioned embodiment of the present invention, whether or not the antitumor effect resulting from the VEGF inhibitor will last for a long period of time in the subject can be predicted with excellent accuracy by using a biological sample that is minimally invasive to the subject.

### BRIEF DESCRIPTION OF THE DRAWINGS

FIG. 1 is a view showing the regimen of VEGF inhibitor (bevacizumab) + FOLFOX therapy implemented in Example 1.
FIG. 2 is an abdominal CT scan image of a patient having an ID No. 4 in Example 1.
FIG. 3 is a thoracic CT scan image of a patient having an ID No. 22 in Example 1.
FIG. 4 is an abdominal CT scan image of a patient having an ID No. 9 in Example 1.
FIG. 5 is a thoracic CT scan image of the patient having an ID No. 9 in Example 1.

### DETAILED DESCRIPTION OF THE INVENTION

In the related art, it is considered that an RAS gene or a protein thereof (RAS protein) in blood is a gene or protein separated from tumor tissue by infiltrating blood vessels, and the status (genotype) of RAS in blood agrees with the status of RAS in tumor tissue. However, contrary to the disclosures in the related art, the status of KRAS in blood does not agree with the status of KRAS in tumor tissue in some cases. In these cases, surprisingly, long-term efficacy of a VEGF inhibitor in the tumor tissue depends more on the status of KRAS in blood than on the status of KRAS in the tumor tissue, and this finding was first acquired by the inventors of the present invention.

A method for predicting long-term efficacy of a VEGF inhibitor of the present invention (hereinafter, referred to as a "method for predicting long-term efficacy according to the present invention" in some cases) is a method for predicting long-term efficacy of a VEGF inhibitor by using the status of RAS in blood as an indicator regardless of the status of RAS in tumor tissue. For a subject who is found to have wild-type RAS in blood and not to have mutant RAS, regardless of whether RAS in tumor tissue is a wild type or a mutant, it is predicted that the antitumor effect resulting from the administration of the VEGF inhibitor is highly likely to last for a long period of time, for example, for a year and a half or longer. Inversely, for a subject who is found to have mutant RAS in blood, it is predicted that long-term efficacy of the VEGF inhibitor will be low and the antitumor effect resulting from the VEGF inhibitor is highly likely to last for only a relatively short period of time.

Actually, as shown in Example 1, which will be described later, there were three cases in which the status of KRAS in serum was found to be a wild type even though a KRAS gene in a primary focus or a metastatic focus was a mutant. In two out of these cases, the efficacy of combination chemotherapy using bevacizumab plus FOLFOX lasted for a long period of time such as a year and a half or longer. In one out of the three cases, in which the efficacy of the bevacizumab + FOLFOX therapy did not last for a long period of time, the tumor recurred four months after the resection of the primary focus. In the patient of this case, a mutant of G12A of the serum KRAS was detected before the resection of the primary focus, and the serum KRAS turned into a wild type after the resection of the primary focus. However, in the following periodical examination, G12A was detected again from the serum.

That is, in a case where the presence of a mutated RAS gene is confirmed in tumor tissue, it is possible to predict that the long-term efficacy of the VEGF inhibitor will be low in a subject who is found to have the mutated RAS gene in circulating DNA (cell-free (cf) DNA) in peripheral blood. Inversely, in a case where the presence of the mutated RAS gene is not confirmed from circulating DNA in peripheral blood, it is possible to predict that the tumor of the subject may be a tumor in which the VEGF inhibitor exhibits long-term efficacy. In this way, the long-term efficacy of the VEGF inhibitor can be predicted with excellent accuracy not by using the status of RAS in tumor tissue but by using the status of RAS in blood as an indicator. This finding was first acquired by the inventors of the present invention.

That is, the method for predicting long-term efficacy according to an embodiment of the present invention is a method for predicting long-term efficacy of a VEGF inhibitor exerted on a tumor of a subject, and includes the steps (a) and (b) described below.

A method for predicting long-term efficacy of a VEGF inhibitor exerted on a tumor of a subject, including: (a) a step of determining whether or not an RAS gene-derived nucleic acid or an RAS protein is present in a blood sample collected from the subject and determining whether the RAS gene-derived nucleic acid in the blood sample is a wild type or a mutant or whether the RAS protein in the blood sample is a wild type or a mutant, and (b) a step in which in a case where the wild-type RAS gene-derived nucleic acid or the wild-type RAS protein is detected in the blood sample in the step (a) and in a case where a mutant RAS gene-derived nucleic acid or a mutant RAS protein is not detected in the blood sample in the step (a), it is determined that an antitumor effect resulting from the VEGF inhibitor is highly likely to last for a long period of time in the subject, and in a case where the mutant RAS gene-derived nucleic acid or the mutant RAS protein is detected in the blood sample in the step (a), it is determined that the antitumor effect resulting from the VEGF inhibitor is unlikely to last for a long period of time in the subject.

RAS detected in the method for predicting long-term efficacy according to an embodiment of the present invention may be HRas, NRas, or KRAS. In the method for predicting long-term efficacy according to an embodiment of the present invention, it is preferable to use the genotype of KRAS as an indicator for determination.

The mutant RAS detected in the method for predicting long-term efficacy according to an embodiment of the present invention includes all forms of mutations such as insertion, inversion, deletion, and point mutation. Unlike the wild-type RAS which is found in a single allele (heterozygous) or in a pair of alleles (homozygous), the mutated RAS genes described above are mutant RAS which can be found in somatic cells or a germline. The somatic cell mutation occurs only in a certain type of tissue such as tumor tissue and is not inherited in the germline. The germline mutation can be found in any body tissue.

The mutant RAS detected in the method for predicting long-term efficacy according to an embodiment of the present invention is preferably mutated RAS accompanied by substitution of one amino acid or two or more amino acids in codons 12, 13, 61, and 146 on exons 2 to 4 of RAS genes. Specifically, examples thereof include mutant RAS having one mutation or two or more mutations selected from the group consisting of G12A, G12C, G12D, G12R, G12S, G12V, G12S2, G13D, G13A, G13S, G13V, G13R, G13C, Q61H, Q61L, Q61R, Q61K, A146G, A146T, and A146V. Table 1 shows the form of nucleic acid (gene) mutation of each mutant. The amino acid sequence of KRAS is represented by SEQ ID NO: 1; the gene sequence including codon 12 on exon 2 of KRAS is represented by SEQ ID NO: 2; the gene sequence including codon 61 on exon 3 of KRAS is represented by SEQ ID NO: 3; and the gene sequence including codon 146 on exon 4 of KRAS is represented by SEQ ID NO: 4.

**[Table 1]**

| Amino acid substitution | Nucleic acid mutation | Exon |
|---|---|---|
| G12A | 5571G > C | 2 |
| G12C | 5570G > T | 2 |
| G12D | 5571G > A | 2 |
| G12R | 5570G > C | 2 |
| G12S | 5570G > A | 2 |
| G12V | 5571G > T | 2 |
| G12S2 | 5570G > T, 5570G > C | 2 |
| G13D | 5574G > A | 2 |
| G13A | 5574G > C | 2 |
| G13V | 5574G > T | 2 |
| G13R | 5573G > C | 2 |
| G13S | 5573G > A | 2 |
| G13C | 5573G > T | 2 |
| Q61H | 23579A > C | 3 |
| Q61L | 23578A > T | 3 |
| Q61R | 23578A > G | 3 |
| Q61K | 23577C > A | 3 |
| A146G | 25293G > C | 4 |
| A146V | 25294C > T | 4 |
| A146T | 25293G > A | 4 |

In the method for predicting long-term efficacy according to an embodiment of the present invention, instead of the status of RAS in tumor tissue, the status of RAS in the blood sample is investigated in the step (a). The blood sample may be peripheral blood, serum, or plasma. Compared to tumor tissue, the blood sample can be collected from a subject in a minimally invasive manner. Therefore, for a subject such as a recurrent tumor patient from whom a biological sample of tumor tissue is not easily collected, the long-term efficacy of the VEGF inhibitor can also be predicted. Furthermore, because the blood sample can be collected from a subject over a long period of time, the method for predicting long-term efficacy according to an embodiment of the present invention is suitable for monitoring whether the tumor has recurred.

As the blood sample, regardless of a primary tumor, a metastatic tumor, and a recurrent tumor, a blood sample collected from a subject having a tumor of an initial stage is preferable. Specifically, the blood sample used in the method for predicting long-term efficacy according to an embodiment of the present invention is preferably a sample in which CEA is equal to or less than 5 ng/mL and a CA-19-9 level is equal to or less than 37.0 U/mL.

In the method for predicting long-term efficacy according to an embodiment of the present invention, the VEGF inhibitor whose long-term efficacy is to be predicted is not particularly limited as long as it is a substance exerting a VEGF inhibitory effect in animals including a human being, and may be an anti-VEGF antibody or TKI. Specific examples of the anti-VEGF antibody include bevacizumab (trade name: Avastine (registered trademark)) and the like. In the method for predicting long-term efficacy according to an embodiment of the present invention, it is preferable to predict the long-term efficacy of one or more VEGF inhibitors among the above VEGF inhibitors. Particularly, it is preferable to predict the long-term efficacy of bevacizumab.

In the method for predicting long-term efficacy according to an embodiment of the present invention, the tumor examined to predict the long-term efficacy of the VEGF inhibitor is not particularly limited as long as it is a VEGF-mediated tumor (cancer), that is, a tumor formed by a process in which VEGF plays a certain role. The tumor includes cancer of the brain, liver, kidney, bladder, breast, stomach, ovary, colorectum, prostate, pancreas, breast, lung, pudendum, thyroid gland, colorectum, esophagus, and liver, sarcoma, glioblastoma, head and neck, leukemia, and malignant lymphatic diseases. More specifically, the tumor can include neuroblastoma, intestinal cancer (for example, rectal cancer, colorectal cancer, familial adenomatous polyposis cancer, and hereditary nonpolyposis colorectal cancer), esophageal cancer, lip cancer, laryngeal cancer, hypopharyngeal cancer, tongue cancer, salivary gland cancer, gastric cancer, adenocarcinoma, thyroid medullary carcinoma, papillary carcinoma of thyroid artery, renal cancer, cancer of renal parenchyma, ovarian cancer, cervical cancer, cancer of corpus uteri, endometrial cancer, choriocarcinoma, pancreatic cancer, prostatic cancer, testis cancer, breast cancer, ureteral cancer, melanoma, brain tumors (for example, glioblastoma, astrocytoma, meningioma, medulloblastoma, and Peripheral neuroectodermal tumour), Hodgkin lymphoma, non-Hodgkin lymphoma, Burkitt lymphoma, acute lymphoblastic leukemia (ALL), chronic lymphocytic leukemia (CLL), acute myeloid leukemia (AML), chronic myeloid leukemia (CML), adult T-cell leukemia, hepatoma, gallbladder cancer, bronchial cancer, small cell lung cancer, non-small cell lung cancer, multiple myeloma, basal cell tumor, tetratoma, retinoblastoma, choroidal melanoma, spermocytoma, rhabdomyosarcoma, craniopharyngeoma, osteosarcoma, chondrosarcoma, myosarcoma, liposarcoma, fibrosarcoma, Ewing's sarcoma, and plasmacytoma. The tumor examined to predict the long-term efficacy in the method for predicting long-term efficacy according to an embodiment of the present invention is preferably one or more tumors selected from the group consisting of colorectal cancer, colon cancer, rectal cancer, lung cancer, liver cancer, breast cancer, ovarian cancer, prostatic cancer, renal cancer, esophageal cancer, head and neck cancer, uterine cancer, and cervical cancer.

In the method for predicting long-term efficacy according to an embodiment of the present invention, the tumor examined to predict the long-term efficacy of the VEGF inhibitor may be a primary focus (primary tumor) or a metastatic focus (metastatic tumor). Furthermore, the tumor may be a recurrent tumor. In addition, the tumor may be present in a plurality of places in the body of the subject.

In the method for predicting long-term efficacy according to an embodiment of the present invention, it is preferable to predict the long-term efficacy of the VEGF inhibitor for a recurrent tumor. For example, by using a blood sample collected from a subject who has undergone surgical resection of a tumor portion in the past or from a subject who has been administered with the VEGF inhibitor in the past, the long-term efficacy of the VEGF inhibitor exerted on a recurrent tumor or a metastatic focus can be predicted. In a case where the subject has been administered with the VEGF inhibitor in the past, it is preferable to use a blood sample collected 60 days after the administration of the VEGF inhibitor. In the therapy using the VEGF inhibitor, in order to implement the therapy while predicting the long-term efficacy of the VEGF inhibitor during the period of the therapy, it is preferable to continuously perform the method for predicting long-term efficacy according to an embodiment of the present invention. The reason is assumed to be as below. Generally, blood collection for tumor marker screening is performed approximately once a month, and CT screening is performed approximately once every three months. Therefore, in order to keep pace therewith, it is preferable to perform the method for predicting long-term efficacy according to an embodiment of the present invention for about every 60 days.

The blood sample used in the method for predicting long-term efficacy according to an embodiment of the present invention may be a sample collected from a subject who has exhibited drug resistance with respect to the VEGF inhibitor in the past.

Even if the subject has exhibited drug resistance with respect to the VEGF inhibitor in the past, in a case where a mutant RAS gene-derived nucleic acid or a protein thereof is not detected from the blood sample of the subject, at a point in time when the blood sample is collected from the subject, it can be determined that the antitumor effect resulting from the VEGF inhibitor is highly likely to last for a long period of time, and that a tumor-shrinking effect is highly likely to last for a long period of time (for example, for a year and a half or longer from the starting point of the administration) by the intake of the VEGF inhibitor. Inversely, in a case where the mutant RAS gene-derived nucleic acid or a protein thereof is detected from the blood sample, at a point in time when the blood sample is collected from the subject, it can be determined that the sensitivity with respect to the VEGF inhibitor is low and that a tumor-shrinking effect is highly unlikely to last even if the subject takes the VEGF inhibitor.

It is also preferable that the method for predicting long-term efficacy according to an embodiment of the present invention be performed using a blood sample collected from a subject rechallenging the VEGF inhibitor. The "subject rechallenging the VEGF inhibitor" means a subject who has been administered with the VEGF inhibitor and then received antitumor therapy different from the administration of the VEGF inhibitor and is going to be administered again with the VEGF inhibitor. Although the administration of the VEGF inhibitor results in resistance in some cases, by investigating in advance the status of RAS in blood before the rechallenge, the long-term efficacy of the VEGF inhibitor in the rechallenge can be predicted. That is, in a case where the mutant RAS gene-derived nucleic acid or a protein thereof is not detected from the blood sample collected from the subject before the rechallenge, it can be determined that the VEGF inhibitor is highly likely to exert efficacy for a long period of time in the subject, and that a tumor-shrinking effect is highly likely to be obtained for a long period of time by the rechallenge to the VEGF inhibitor. Inversely, in a case where the mutant RAS gene-derived nucleic acid or a protein thereof is detected from the blood sample, it can be determined that the long-term efficacy of the VEGF inhibitor is low in the subject, and that a tumor-shrinking effect is highly unlikely to last for a long period of time even if the subject rechallenges the VEGF inhibitor.

As the antitumor therapy different from the administration of the VEGF inhibitor that is implemented before the rechallenge to the VEGF inhibitor, those appropriately selected from known antitumor therapies according to the pathologic conditions and the like of the subject can be used. Specific examples of the different antitumor therapy include radiotherapy, medication therapy using a chemotherapy agent, medication therapy using a molecular-targeted drug of a type different from the VEGF inhibitor which has already been administered, and the like. The different antitumor therapy may be one antitumor therapy or a combined modality therapy composed of two or more antitumor therapies. For example, for the method for predicting long-term efficacy according to an embodiment of the present invention, a combined modality therapy composed of medication therapy using a molecular-targeted drug and medication therapy using a chemotherapy agent is preferable.

The chemotherapy agent is not limited and can be a cytotoxic compound or a cell division inhibitory compound. Specifically, the chemotherapy agent includes (i) an antimetabolite such as fluorouracil, cytarabine, fludarabine, 5-fluoro-2'-deoxyuridine, gemcitabine, hydroxyurea, or methotrexate; (ii) a DNA-fragmenting agent such as bleomycin; (iii) a DNA-crosslinking agent such as chlorambucil, cisplatin, cyclophosphamide, or nitrogen mustard; (iv) an intercalating agent such as adriamycin (doxorubicin) or mitoxantrone; (v) a protein synthesis inhibitor such as L-asparaginase, cycloheximide, puromycin, or diphtheria toxin; (vi) topoisomerase I poison such as camptothecin or topotecan; (vii) topoisomerase II poison such as etoposide (VP-16) or teniposide; (viii) a microtubule-associated agent such as colcemid, colchicine, paclitexel, vinblastine, or vincristine; (ix) a kinase inhibitor such as flavopiridol, staurosporine, STI571 (CPG57148B), or UCN-01 (7-hydroxystaurosporine); (x) various investigational drugs such as thioplatin, PS-341, phenylbutyrate, ET 18-OCH3, and farnesyltransferase inhibitors (L-739749 and L-744832); polyphenols such as quercetin, resveratrol, piceatannol, Epigallocatechin gallate, Theaflavins, flavanols, procyanidins, and betulinic acid and derivatives thereof; (xi) hormones such as glucocorticoid or fenretinide; and (xii) antihormones such as tamoxifen, finasteride, or an LHRH antagonist. Furthermore, the chemotherapy agent includes folinic acid, oxaliplatin, irinotecan, daunorubicin, taxotere, and mitomycin C. In the different antitumor therapy described above, one chemotherapy agent among these may be used singly, or two or more thereof may be used concurrently.

In a case where the method for predicting long-term efficacy according to an embodiment of the present invention is performed using the blood sample collected from a subject who has been administered with the VEGF inhibitor and then received the medication therapy using the chemotherapy agent and is going to rechallenge the VEGF inhibitor, the chemotherapy agent is preferably one or more agents selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecine, topotecan, teniposide, colcemid, colchicine, paclitexel, vinblastine, vincristine, and tamoxifen.

Specific examples of the aforementioned molecular-targeted drug include one or more drugs selected from the group consisting of cetuximab, panitumumab, bevacizumab, gefitinib, erlotinib, regorafenib, crizotinib, sunitinib, sorafenib, everolimus, trastuzumab, lapatinib, and rituximab.

It is also preferable that the method for predicting long-term efficacy according to an embodiment of the present invention be performed for a blood sample collected from a subject who has been administered with an EGFR inhibitor in the past. For example, in a case where resistance to an anti-EGFR antibody drug is observed and thus the VEGF inhibitor therapy is used as a different therapy, the blood sample described above is used. The blood sample may also be collected from a subject who has been administered with the EGFR inhibitor and then received antitumor therapy different from the administration of the EGFR inhibitor and is going to receive medication therapy using the VEGF inhibitor.

As the different antitumor therapy, those appropriately selected according to the pathologic conditions and the like of the subject can be used. Specific examples thereof include radiotherapy, medication therapy using a chemotherapy agent, medication therapy using a molecular-targeted drug of a type different from the EGFR inhibitor which has already been administered, and the like. Examples of the chemotherapy agent or the molecular-targeted drug include the chemotherapy agents or the molecular-targeted drugs described above.

In the step (a) described above, the status of RAS in the blood sample may be determined at a protein level or at a nucleic acid level (genome DNA or mRNA). In the method for predicting long-term efficacy according to an embodiment of the present invention, it is preferable to measure the RAS gene-derived nucleic acid, since RAS can be detected with high sensitivity. Specifically, it is preferable that whether RAS is present in the blood sample and whether RAS is a wild type or a mutant are determined by investigating whether or not the wild-type and mutant RAS gene-derived nucleic acids are detected from circulating DNA in the blood sample. Examples of the RAS gene-derived nucleic acids include full-length genome DNA of an RAS gene or a portion thereof, full-length mRNA of an RAS gene or a portion thereof, cDNA obtained by using the full-length mRNA or a portion thereof as a template, an amplification product obtained by artificially amplifying the above nucleic acids through a polymerase chain reaction (PCR), and the like.

The detection of the RAS gene-derived nucleic acid in the blood sample and the determination of the genotype of the detected RAS gene-derived nucleic acid can be performed by a common method. In the present invention, in view of prediction accuracy, it is preferable to perform the detection under measurement conditions which make it possible to detect 0.1 % mutant.

For example, the presence or status of RAS in the blood sample can be determined by detecting the RAS gene-derived nucleic acid contained in the blood sample by using digital PCR. Particularly, by using the technique of a droplet digital PCR (ddPCR) from Bio-Rad Laboratories, Inc. (Hindson et al., Analytical Chemistry, 2011, vol. 83(22), pp.8604-8610), the RAS gene-derived nucleic acid can be detected with high sensitivity. The greater the number of droplets, the higher the analysis accuracy. In order to assure the performance of detecting 0.01% mutation, 10,000 droplets are required for detecting a single mutation. Therefore, it is preferable to specify the concentration of a surfactant in a master mix of PCR. For example, the final concentration of ethylene glycol or glycerol used as a conservation solution of a DNA elongation enzyme or the like is preferably equal to or less than 0.15%, and the final concentration of Triton-X is preferably equal to or less than 0.0003%. If the final concentration of the aforementioned surfactants is equal to or higher than the above, an emulsion number resulting from the droplets greatly decreases, and accordingly, it is difficult to detect mutation with high sensitivity.

It is also preferable to use a method in which the nucleic acid and nucleic acid fragments obtained from the blood sample are subjected to a first PCR session performed for 15 to 50 cycles so as to increase the absolute amount of allele copies of the mutant RAS that may be contained in the nucleic acids, the product is then diluted such that the number of the copies becomes about 106, and thereafter, a known mutation detection method is performed. According to this method, even if the number of types of mutations is increased so as to increase the total number of mutants present in the reaction system, it is possible to reduce a likelihood that mutant alleles may be physically nonexistent and thus failed to be detected. This method may be combined with digital PCR described above.

As another method, for example, there is a method in which a fragment including a region encoding the mutation site in the RAS gene is amplified by PCR or the like using the nucleic acid in the blood sample as a template; the amplification product is then brought into contact with a probe which can be specifically hybridized with a certain genotype of KRAS; and whether or not an aggregate is formed is detected with high sensitivity. It is also preferable that the diluted amplification product may be subjected to emulsion PCR before the hybridization is performed.

The aforementioned probe is labeled with, for example, a radioisotope (3H, 32P, 33P, or the like), a fluorescent agent (rhodamine or fluorescein), or a color-developing agent such that it can be detected. The probe may be an antisense oligomer such as PNA, morpholino-phosphoramidates, or LNA. The base length of the probe can consist of about 8 to 100 nucleotides, about 10 to 75 nucleotides, about 15 to 50 nucleotides, or about 20 to 30 nucleotides.

The presence of RAS or the status thereof in the blood sample can be analyzed using an Invader method (Michael Oliver, Mutation Research 573:103-110, 2005). In the Invader (registered trademark) method, an allele probe and an invader oligo are hybridized with double-strand DNA, which is prepared by PCR or the like, or mRNA so as to partially form a triplex base. Herein, a portion of the 5'-terminal of the allele probe is designed such that it has a sequence (flap portion) non-complementary to the double-strand DNA or the mRNA. In contrast, the invader oligo has a sequence totally complementary to the double-strand DNA or the mRNA. Two types of allele probes are designed such that they become complementary to each of the wild type and the mutant, and competitively hybridized with the double-strand DNA or the mRNA. When totally complementary hybridization occurs, flap end nuclease recognizes the portion that has turned into a triplex base and cleaves a flap portion of the allele probe. The flap portion of the cleaved allele probe is hybridized with a self-complementary FRET cassette present in the same reaction system. At this time, a portion which will become a triplex base is formed, the flap end nuclease cleaves the mutation of interest, and as a result, a fluorescence-labeled DNA fragment in the FRET cassette is liberated. The DNA fragment fluoresces because of being separated from a quencher in the FRET cassette. Theoretically, once the flap portion of the allele probe is cleaved, it can be hybridized again with another FRET cassette, and this leads to signal amplification. The invader method is a technique that can detect mutation with extremely high sensitivity in the manner described above.

The ligase chain reaction known in the related art can be used to amplify fragments including a region encoding the mutation site in the RAS gene (for example, see Wu, et al., Genomics, 1989, vol. 4, pp.560-569). Furthermore, a technique known as allele-specific PCR can be used (for example, see Ruano and Kidd, Nucleic Acids Research, 1989, vol. 17, pp.8392). According to this technique, a primer hybridized with the 3'-terminal of a specific RAS mutation is used. In a case where there is no specific RAS mutation, an amplification product is not observed. In addition, it is possible to use the Amplification Refractory Mutation System (ARMS) (for example, see European Patent Application, Publication No. 0332435 and Newton et al., Nucleic Acids Research, 1989, vol. 17, pp. 7).

In order to detect the RAS gene-derived nucleic acid in the blood sample or to determine the genotype of the detected RAS gene-derived nucleic acid, it is possible to use a method other than those used for detecting gene mutation or for detecting insertion and deletion of genes. Specific examples of the method include a method of directly determining the base sequence of genome DNA or mRNA of the RAS gene in the blood sample or the base sequence of amplification products thereof by using a sequence analysis method based on a Sanger method. Furthermore, the base sequence can be determined through PCR. In addition, in order to score the alteration or insertion of alleles in polymorphic fragments, a restriction fragment length polymorphism (RFLP) probe for a gene or the surrounding marker gene can be used. For detecting a mutant caused by the change in base of the allele, single-strand conformation polymorphism (SSCP) analysis can be used (Orita et al., Proceedings of the National Academy of Science, USA, 1989, vol. 86, p.2766-2770 and Genomics, 1989, vol. 5, p. 874-879).

By preparing the reagents and the like which are used for detecting the RAS gene-derived nucleic acid in the blood sample or for determining the genotype of the detected RAS gene-derived nucleic acid in the form of a kit, the method for predicting long-term efficacy according to an embodiment of the present invention can be performed in a more simplified manner. Examples of the reagents include a reagent for extracting a nucleic acid from the blood sample, an enzyme such as polymerase or ligase, a probe or primer which can be specifically hybridized with a certain genotype of RAS (oligonucleotide which is specifically hybridized with a mutation site of the RAS gene or with a site adjacent thereto), and the like. The kit may include protocol regarding the method for detecting the RAS gene-derived nucleic acid in the blood sample or the method for determining the genotype thereof, a document describing the instructions regarding the criteria used for determining the long-term efficacy of the VEGF inhibitor from the result of the obtained genotype (status) of RAS, and the like.

The method for predicting long-term efficacy according to an embodiment of the present invention can provide important information for determining whether or not the VEGF inhibitor will be administered. That is, the method for predicting long-term efficacy according to an embodiment of the present invention can provide useful information to clinicians, and based on the information obtained by the method, the clinicians can determine an appropriate therapeutic method.

### Examples

Hereinafter, the present invention will be specifically described by illustrating examples, but the present invention is not limited to the following examples.

### [Example 1]

For 23 patients with recurrent colorectal cancer who had undergone surgical resection of primary foci, a nonsynonymous mutation accompanied by amino acid substitution of KRAS contained in the primary foci, metastatic foci, and serum prepared from their blood collected after confirming the metastatic foci was investigated.

### (Clinical sample)

From the patients with recurrent colorectal cancer, 6 mL of peripheral blood was collected before or after the surgical resection of the primary foci and subjected to centrifugation (3,000 rpm, 10 min), thereby obtaining serum components. From a patient having an ID No. 9, 6 mL of peripheral blood was collected again after the surgical resection of the metastatic focus, and the serum components were obtained in the same manner as described above (Sample No. 16). Furthermore, formalin-fixed and paraffin-embedded (FFPE) slices from the primary and metastatic foci of some of the patients were also used as test samples. The present test was approved by the ethics committee in NIPPON MEDICAL SCHOOL HOSPITAL and performed after obtaining informed consent including the present research from all of the patients. Table 2 shows the information on the patients participating in the present test. In Table 2, "-" in the column of "Metastatic focus" means that the metastatic focus of the patient has not yet been confirmed. Furthermore, in Table 2, the column of "Molecular-targeted drug" shows a molecular-targeted drug administered through a combined modality therapy using the molecular-targeted drug plus FOLFOX that was implemented after the surgical resection of the metastatic focus (here, regarding Sample Nos. 8 and 15, the molecular-targeted drug was administered before the surgical resection of the primary focus). That is, in the same column, "b-mab" indicates a combined modality therapy using bevacizumab + FOLFOX, and "C-Mab" indicates a combined modality therapy using cetuximab + FOLFOX. In Table 2, the column of "Chemotherapy" shows the state of chemotherapy implemented at a point in time when the molecular-targeted drug is administered after the surgical resection of the metastatic focus (here, for Sample Nos. 20 and 21, a point in time when the molecular-targeted drug is administered after the surgical resection of the primary focus, and for Sample No. 16, a point in time when the blood sample is collected). More specifically, "N/A" means a state where the administration of the drug has not yet been decided but the patient is likely to be administered with the drug in the future; "Before therapy" means that the therapy is scheduled; "Under therapy" means a state where the chemotherapy exerts efficacy and the administration of the drug continues; "After therapy" means that the administration of the drug has been stopped at that point in time even though the chemotherapy exerted efficacy; and "After termination of therapy" means a state where the chemotherapy does not exert efficacy any longer (that is, a state where the patient receives palliative care).

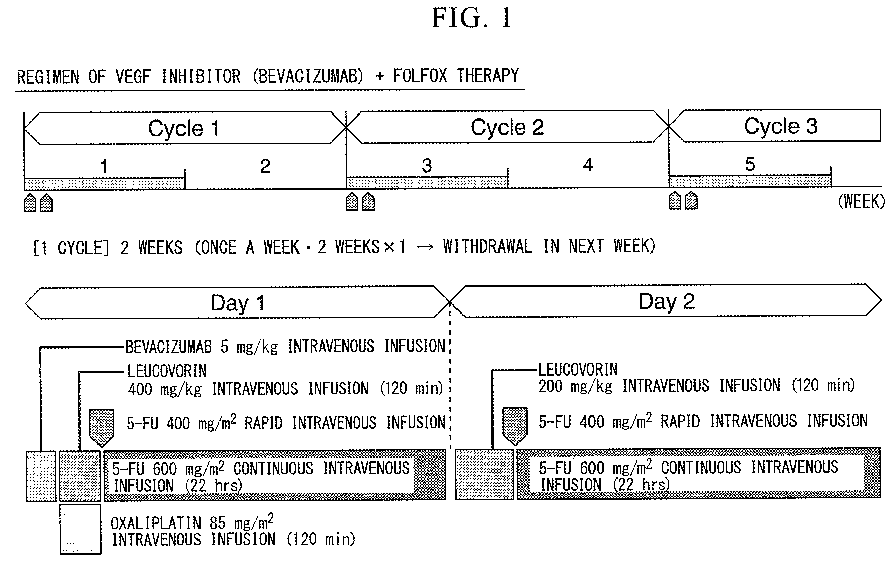

To the patients having ID Nos. 1 to 5, 9 to 11, 16, 17, and 19 to 23 whose primary foci had mutant KRAS, chemotherapy using bevacizumab, which is an anti-VEGF inhibitor, and FOLFOX (fluorouracil·folinic acid·oxaliplatin) was provided. Bevacizumab was administered by intravenous drip infusion every 2 weeks at a dosage of 5 mg/kg and an infusion rate of 0.5 mg/kg/min (5 mg/kg/10 min). The initial administration time was 90 minutes. Due to tolerability, the administration time was decreased to 60 minutes for a second dose and to 30 minutes after a third dose. In the chemotherapy using FOLFOX, folinic acid (leucovorin) at a dosage of 400 mg/body and oxaliplatin at a dosage of 145 or 140 mg/body were administered by intravenous drip infusion for 2 hours, and fluorouracil (5-FU) was administered at a dosage of 675 or 650 mg/body by rapid intravenous drip infusion and then at a dosage of 4,100 mg/body by continuous intravenous drip infusion for 22 hours. The administration regimen is shown in FIG. 1.

**[Table 2]**

| Sample No. | Patient ID | Gender | Age | Primary focus | Metastatic focus | Chemotherapy | Molecular-targeted drug |
|---|---|---|---|---|---|---|---|
| 1 | 1 | Male | 67 | Ascending colon cancer | Peritoneal seeding | Under therapy | b-mab |
| 2 | 2 | Male | 70 | Ascending colon cancer | Liver metastasis | After termination of therapy | b-mab |
| 3 | 3 | Male | 67 | Rectal cancer | Liver/lung metastasis | Under therapy | b-mab |
| 4 | 4 | Male | 69 | Rectal cancer | Lung metastasis | After termination of therapy | b-mab |
| 5 | 5 | Female | 68 | Transverse colon cancer | Peritoneal seeding | After termination of therapy | b-mab |
| 6 | 6 | Female | 50 | Transverse colon cancer | Liver metastasis | Under therapy | c-mab |
| 7 | 7 | Male | 54 | Rectal cancer | Liver metastasis | Under therapy | c-mab |
| 8 | 8 | Male | 71 | Rectal cancer | Lung metastasis | Under therapy | c-mab |
| 9 | 9 | Female | 66 | Rectal cancer | Peritoneal seeding | N/A | b-mab |
| 10 | 10 | Male | 78 | Rectal cancer | Liver/lung metastasis | Under therapy | b-mab |
| 11 | 11 | Female | 70 | Sigmoid colon cancer | Liver/lung metastasis | Under therapy | c-mab |
| 12 | 12 | Female | 79 | Rectal cancer | Liver/lung metastasis | Under therapy | c-mab |
| 13 | 13 | Female | 50 | Transverse colon cancer | Liver/lung metastasis | Under therapy | c-mab |
| 14 | 14 | Female | 60 | Rectal cancer | Liver/lung metastasis | Under therapy | c-mab |
| 15 | 9 | Female | 66 | Rectal cancer | Same as patient ID No. 9 | After therapy | b-mab |
| 16 | 15 | Female | 69 | Rectal cancer | Peritoneal seeding | N/A | b-mab |
| 17 | 16 | Male | 66 | Ascending colon cancer | Liver metastasis | Under therapy | c-mab |
| 18 | 17 | Male | 82 | Rectal cancer | Peritoneal seeding and lung metastasis | After therapy | b-mab |
| 19 | 18 | Female | 81 | Ascending colon cancer | - | N/A | b-mab |
| 20 | 19 | Male | 69 | Rectal cancer | - | Before therapy | b-mab |
| 21 | 20 | Female | 88 | Rectal cancer | Liver metastasis | N/A | b-mab |
| 22 | 21 | Female | 81 | Ascending colon cancer | Peritoneal seeding | N/A | b-mab |
| 23 | 9 | Female | 66 | Rectal cancer | Same as patient ID No. 9 | After therapy | b-mab |
| 24 | 22 | Male | 66 | Colon cancer | Liver/lung metastasis | After therapy | b-mab |

### (Measurement of CEA and CA-19-9 in serum)

By a chemiluminescence enzyme immunoassay (CLEIA method), CEA and CA-19-9 in serum were measured. The measurement results are shown in Table 3.

### (Isolation and purification of cell-free (cf) DNA from serum)

cfDNA was isolated and purified from serum by using a QIAamp Circulating Nucleic Acid Kit (QIAGEN). The amount of serum samples used in the kit varied with the patients and was 2 mL to 4 mL. The step of isolating and purifying DNA was performed according to the instructions attached to the kit. The final elution from a spin column was performed using 50 µL of TE buffer solution.

### (Isolation and purification of DNA from FFPE slice)

DNA was isolated and purified from FFPE slices by using a QIAamp DNA FFPE Tissue Kit (QIAGEN). For each sample, three FFPE slices sliced at 10 µm were used. The step of isolating and purifying DNA was performed according to the instructions attached to the kit. The final elution from a spin column was performed using 100 µL of a TE buffer solution.

### (DNA quantification)

The DNA isolated and purified from the cfDNA and the FFPE slices were quantified by using Quant-iT (registered trademark) PicoGreen (registered trademark) dsDNA Reagent and Kits (Invitrogen). All of the samples to be measured were used after the isolated DNA was diluted 20x with a TE buffer solution. As a fluorometer, SAFIRA (TECAN TRADING AG) was used.

### (Direct sequencing)

The KRAS base sequence in surgical samples of primary and metastatic foci and in serum was analyzed by direct sequencing. As primer sequences for direct sequencing of KRAS, KRAS (forward): 5'-GAATGGTCCTGCACCAGTAA-3' (SEQ ID NO: 5) and KRAS (reverse): 5'-GTGTGACATGTTCTAATATAGTCA-3' (SEQ ID NO: 6) were used. Each PCR product had a length of 214 bp. PCR was performed under conditions below. After pre-denaturation was performed for 10 minutes at 95°C, PCR was performed for 40 cycles each consisting of a cycle conducted for 20 seconds at 94°C, 20 seconds at 60°C, and 30 seconds at 72°C. Then, an elongation reaction was performed for 10 minutes at 72°C. The sequence analysis was performed using ABI 3730 (Applied Biosystems Inc, Foster City, CA) by means of cycle sequencing based on a Bigdye terminator method.

The results are shown in Table 3. In the column of "Serum" of Table 3, "preoperative" and "postoperative" listed at the end of the genotype mean that it is a genotype in serum collected before or after the surgical resection of the primary focus. Furthermore, "-" in the column of "Metastatic focus" of Table 3 means that the genotype of KRAS in the metastatic focus has not been analyzed.

**[Table 3]**

| Sample No. | Patient ID | Primary focus | Metastatic focus | Serum | Serum amount (mL) | cfDNA concentration (ng/µL) | CEA concentration (ng/mL) | CA19-9 level (U/mL) |
|---|---|---|---|---|---|---|---|---|
| 1 | 1 | G12V | - | G12V (postoperative) | 2 | 0.61 | 7.8 | 8.6 |
| 2 | 2 | G12D | - | G12D (postoperative) | 2 | 0.64 | 26.5 | 3726.0 |
| 3 | 3 | G12D | - | G12D (postoperative) | 2 | 0.13 | 231.0 | 1991.2 |
| 4 | 4 | G12V | - | wild (postoperative) | 2 | 0.49 | 3.5 | 9.2 |
| 5 | 5 | G13D | - | G13D (postoperative) | 2 | 0.32 | 3.5 | 3.9 |
| 6 | 6 | wild | - | wild (postoperative) | 2 | 4.70 | 1234.7 | 2.0 |
| 7 | 7 | wild | - | wild (postoperative) | 2 | 3.50 | 2.3 | 11.9 |
| 8 | 8 | wild | wild | G13D (postoperative) | 2 | 1.30 | 9.7 | 25.9 |
| 9 | 9 | G12A | - | G12A (preoperative) | 2 | 1.10 | 2.0 | 10.3 |
| 10 | 10 | G12D | - | G12D (postoperative) | 1.8 | 3.40 | 61.5 | 44.8 |
| 11 | 11 | wild | - | wild (postoperative) | 2 | 34.40 | 19.7 | 85.2 |
| 12 | 12 | wild | - | wild (postoperative) | 2 | 0.23 | 2.3 | 4.4 |
| 13 | 13 | wild | - | wild (postoperative) | 2 | 40.40 | 1158.6 | 2.0 |
| 14 | 14 | wild | wild | G13D (postoperative) | 2 | 1.80 | 9.6 | 45.1 |
| 15 | 9 | G12A | - | wild (postoperative 1) | 3 | 0.60 | 1.5 | 3.4 |
| 16 | 15 | G12V | - | G12V (postoperative) | 3 | 0.80 | 55.2 | 38.7 |
| 17 | 16 | wild | - | wild (postoperative) | 2 | 1.60 | 22.3 | 169.8 |
| 18 | 17 | G12V | - | G12V (postoperative) | 3.5 | 21.50 | 467.0 | 636.6 |
| 19 | 18 | wild | - | wild (preoperative) | 4 | 2.20 | 2.2 | 2.0 |
| 20 | 19 | wild | - | wild (preoperative) | 2 | 1.53 | 79.4 | 35.8 |
| 21 | 20 | G12V | - | G12V (postoperative) | 4 | 22.40 | 433.7 | 8.1 |
| 22 | 21 | wild | - | wild (postoperative) | 3 | 2.05 | n.d. | n.d. |
| 23 | 9 | G12A | - | G12A (postoperative 2) | 3 | n.d. | n.d. | n.d. |
| 24 | 22 | G12D | - | wild (postoperative) | 3 | n.d. | n.d. | n.d. |

The rate of concordance between the KRAS gene mutation of the surgical sample from the primary focus and the KRAS gene mutation in cfDNA was 81.8%. Table 4 shows the correlation between the KRAS gene mutation in the primary focus and the KRAS gene mutation in the cfDNA. In Table 4, "pDNA" means DNA extracted from the primary focus. For calculating a Cohen's Kappa coefficient κ, P (concordance rate) and Pe (a rate of concordance obtained in a case where results obtained from two samples fortuitously coincide with each other) were substituted into □ =(P - Pe)/(1 - Pe), and as a result, the value of κ equaled 0.58.

**[Table 4]**

| | pDNA | | |
|---|---|---|---|
| cfDNA | Mutant type | Wild type | Total |
| Mutant type | 9 | 2 | 11 |
| Wild type | 2 | 9 | 11 |
| Total | 11 | 11 | 22 |

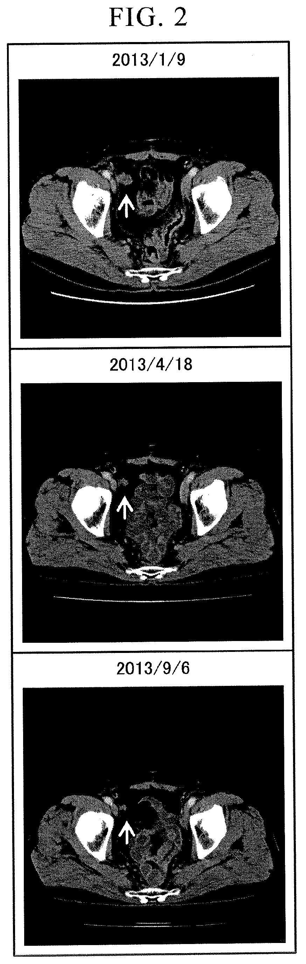

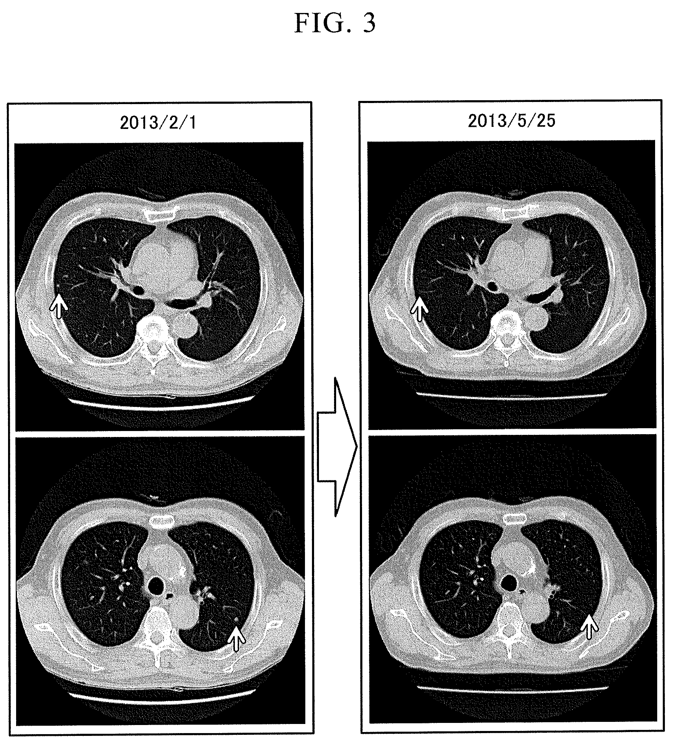

It is noteworthy that among three cases of patients having ID Nos. 4, 9 (Sample No. 15), and 22 (Sample No. 24) in which the status of KRAS in the primary focus was a mutant while the status of KRAS in serum was a wild type, the combined modality therapy using bevacizumab + FOLFOX exerted a marked efficacy for a long period of time in the cases of the patients having ID Nos. 4 and 22. In those two cases, a state of stable disease (SD; a tumor shrinkage rate of less than 30% or a tumor expansion rate of less than 20%) was maintained for a year and a half or longer. The tumor shrinkage rate (shrinking effect) was calculated from the diameter of the tumor in a CT scan image. In a case where the tumor was present in multiple points, the tumor shrinkage rate was calculated by summing up the diameters thereof. A history of therapy and examination and CT scan images (captured on January 9, April 18, and September 6 in 2013) of a patient having an ID No. 4 are shown in Table 5 and FIG. 2 respectively. In addition, a history of therapy and examination and CT scan images (captured on February 1 and May 25 in 2013) of a patient having an ID No. 22 are shown in Table 6 and FIG. 3 respectively. The arrow in FIG. 2 shows a tumor site where the shrinking effect was observed, and the arrow in FIG. 3 shows a disappeared tumor site.

**[Table 5]**

| History of therapy and examination of patient having ID No. 4 | | | | |
|---|---|---|---|---|
| Primary focus | Thorax | Abdomen | FOLFOX/Bev | Serum test |
| G12V | | 2013/1/9 | | |
| | 2013/1/16 No lung metastasis | Lymph node metastasis 22 x 12 mm | | 2013/1/30 wild |
| | | | 2013/2/27 | |
| | | | 2013/3/13 | |
| | | | 2013/3/27 | |
| | | | 2013/4/10 | |
| | | 2013/4/18 | 2013/4/24 | |
| | 2013/4/20 No lung metastasis | Lymph node metastasis 15 x 10 mm (shrunk by 32%) | | |
| | | | 2013/5/8 | |
| | | | 2013/5/22 | |
| | | | 2013/6/5 | |
| | | | 2013/6/19 | |
| | | | 2013/7/3 | |
| | | | 2013/7/24 | |
| | | | 2013/8/21 | |
| | 2013/9/2 No lung metastasis | 2013/9/6 | 2013/9/4 | |
| | | Lymph node metastasis 9 x 6 mm (shrunk by 59%) | | |
| | | | 2013/9/18 | |

**[Table 6]**

| History of therapy and examination of patient having ID No. 22 | | | | |
|---|---|---|---|---|
| Primary focus | Thorax | Abdomen | FOLFOXBev | Serum test |
| G12D | 2013/2/1 | | | |
| | Lung metastasis in 2 points | | | |
| | | | 2013/3/15 | 2013/3/18 KRAS wild |
| 2012/3/25 Colectomy | | | 2013/3/28 | |
| | | | 2013/4/10 | |
| | | | 2001/4/25 | |
| | | | 2013/5/9 | |
| 2013/5/25 Hepatectomy | 2013/5/25 | | | |
| | Disappearance of lung metastasis | | 2013/5/23 | |
| | | | 2013/6/5 | |
| | | | 2013/6/20 | |
| | | | 2013/7/3 | |
| | | | 2013/7/17 | |
| | | | 2013/7/31 | |
| | | | 2013/8/14 | |
| | | | 2013/8/28 | |
| | | | 2013/9/11 | |

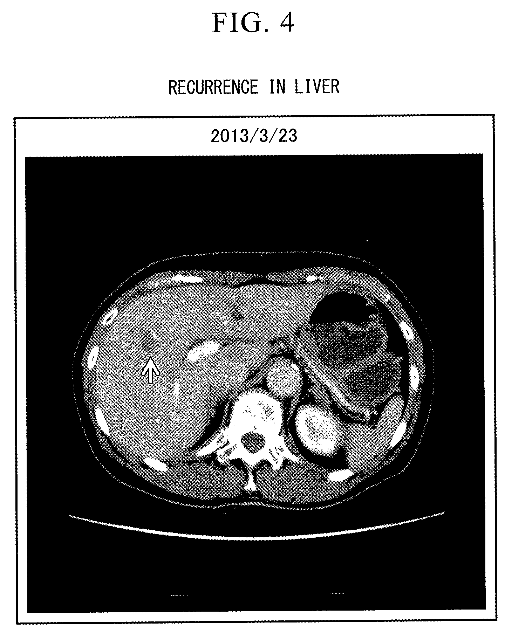

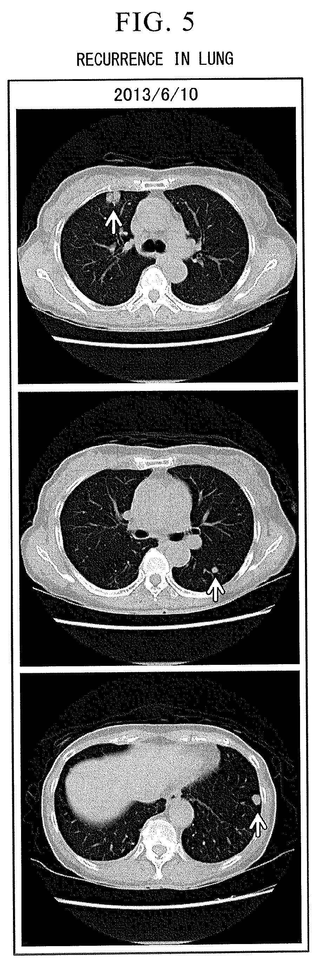

From the circulating DNA of the patient having an ID No. 9 that was obtained before the resection of the primary focus, ultimately the same KRAS gene mutation (G12A) as in the primary focus was observed, while G12A was not detected from the circulating DNA of the same patient that was obtained after the resection of the primary focus. However, G12A was then detected from the serum KRAS, and the tumor recurred after 4 months. That is, in conclusion, the efficacy of bevacizumab was extremely poor. A history of therapy and examination and CT scan images (abdomen: captured on March 23 in 2013, thorax: captured on June 10 in 2013) of a patient having an ID No. 9 are shown in Table 7 and FIGS. 4 and 5 respectively. The arrow in FIGS. 4 and 5 shows a tumor site where the recurrence was confirmed. The results shown in the table and figures imply that the detection of the mutation of the KRAS gene from cfDNA in serum or plasma can be applied to the prognosis of cancer patients or the recurrence diagnosis. Furthermore, by observing cfDNA in peripheral blood before a cancer marker such as CEA or CA-19-9 responds, early diagnosis can be made.

**[Table 7]**

| History of therapy and examination of patient having ID No. 9 | | | | |
|---|---|---|---|---|
| Primary focus | Thorax | Abdomen | FOLFOX/Bev | Serum test |
| | | | Preoperative chemotherapy | |
| | | | 2012/4/27 | |
| | | | 2012/5/10 | |
| | | | 2012/5/24 | |
| | | | 2012/6/7 | |
| | | | 2012/7/4 | |
| | | | 2012/8/1 | |
| | | | | 2012/8/23 |
| | | | | G12A (sample9) |
| 2012/9/10 | | | | |
| Rectectomy G12A | | | | |
| | | | Postoperative chemotherapy | |
| | | | 2012/10/31 | |
| | | | 2012/11/14 | |
| | | | 2012/11/28 | |
| | | | 2012/12/12 | 2012/12/12 |
| | | | | wild (sample 15) |
| | | | 2012/12/26 | |
| | | | 2013/1/9 | |
| | | 2013/3/23 | | |
| | | Liver metastasis | | |
| | | 2.0 cm x 1 | | |
| | | | | 2013/5/10 |
| | | | | G12A (sample 23) |
| | 2013/6/10 | | | |
| | Lung metastasis | | | |
| | 2.0 cm x 1 | | | |
| | 1.0 cm x 1 | | | |
| | 0.8 cm x 1 | | | |

From cfDNA of patients having ID Nos. 8 and 14 whose primary and metastatic foci were wild types, G13D was detected. Furthermore, the administration of an EGFR inhibitor, cetuximab, into a patient having an ID No. 8 did not bring about the tumor-shrinking effect. These results show that the status of the gene mutation of KRAS in tumor tissue does not necessarily become a factor for predicting the therapeutic effect of the EGFR inhibitor. Meanwhile, the CT scan results showed that the tumor shrunk by equal to or more than 20% in patients having ID Nos. 4 and 22.

In the clinical analysis performed on the patients with recurrent colorectal cancer in Example 1, in all of the patients who were observed to have a mutated KRAS gene in the primary focus, the mutated KRAS gene was also observed from their serum collected at the time of recurrence diagnosis. The result shows that it is useful to identify the circulating mutated KRAS gene for making a recurrence diagnosis after the resection of the primary focus, and the presence of a recurrent tumor can be confirmed in early stages (depending on the patient, earlier than a case where the existing biomarker such as CEA or CA19-9 is used) by investigating the status of KRAS in blood over time for subjects who have received therapy for the primary focus.

### INDUSTRIAL APPLICABILITY

The method for predicting long-term efficacy according to the present invention uses peripheral blood. Therefore, long-term efficacy of a VEGF inhibitor can be predicted with high accuracy in a minimally invasive manner, without performing the resection of a primary focus or collecting a biopsy sample. It is considered that due to the minimal invasiveness and excellent accuracy, the method for predicting long-term efficacy according to the present invention will become widespread as a test method replacing a fecal occult blood test used in cancer screening.

## Claims

1. A method for predicting long-term efficacy of a VEGF inhibitor exerted on a tumor of a subject, comprising:
determining whether or not an RAS gene-derived nucleic acid or an RAS protein is present in a blood sample collected from the subject;
determining whether the RAS gene-derived nucleic acid in the blood sample is a wild type or a mutant, or determining whether the RAS protein in the blood sample is a wild type or a mutant;
determining that an antitumor effect resulting from the VEGF inhibitor is highly likely to last for a long period of time in the subject in a case where a wild-type RAS gene-derived nucleic acid or a wild-type RAS protein is detected in the blood sample and a mutant RAS gene-derived nucleic acid or a mutant RAS protein is not detected in the blood sample; and
determining that the antitumor effect resulting from the VEGF inhibitor is unlikely to last for a long period of time in the subject in a case where the mutant RAS gene-derived nucleic acid or the mutant RAS protein is detected in the blood sample.

2. The method for predicting long-term efficacy of a VEGF inhibitor according to Claim 1, wherein
the subject has undergone surgical resection of a tumor portion or has been administered with a VEGF inhibitor in the past.

3. The method for predicting long-term efficacy of a VEGF inhibitor according to Claim 1 or 2, wherein
the RAS is KRas, HRas, or NRAS.

4. The method for predicting long-term efficacy of a VEGF inhibitor according to any one of Claims 1 to 3, wherein
the subject has been administered with a drug other than the VEGF inhibitor.

5. The method for predicting long-term efficacy of a VEGF inhibitor according to any one of Claims 1 to 3, wherein
the subject is a tumor patient who has been administered with the VEGF inhibitor and then received antitumor therapy different from the administration of the VEGF inhibitor, and
the blood sample is collected before the tumor patient is going to be administered again with the VEGF inhibitor.

6. The method for predicting long-term efficacy of a VEGF inhibitor according to Claim 5, wherein
the antitumor therapy is medication therapy using a chemotherapy agent.

7. The method for predicting long-term efficacy of a VEGF inhibitor according to Claim 6, wherein
the chemotherapy agent is one or more agents selected from the group consisting of fluorouracil, folinic acid, oxaliplatin, irinotecan, cytarabine, fludarabine, gemcitabine, hydroxyurea, methotrexate, bleomycin, chlorambucil, cisplatin, cyclophosphamide, doxorubicin, mitoxantrone, camptothecine, topotecan, teniposide, colcemid, colchicine, paclitaxel, vinblastine, vincristine, and tamoxifen.

8. The method for predicting long-term efficacy of a VEGF inhibitor according to Claim 5, wherein
the antitumor therapy is radiotherapy.

9. The method for predicting long-term efficacy of a VEGF inhibitor according to Claim 5, wherein
the antitumor therapy is medication therapy using a molecular-targeted drug different type from the VEGF inhibitor which has already been administered to the subject.

10. The method for predicting long-term efficacy of a VEGF inhibitor according to Claim 9, wherein
the molecular-targeted drug is one or more drugs selected from the group consisting of cetuximab, panitumumab, bevacizumab, gefitinib, erlotinib, regorafenib, crizotinib, sunitinib, sorafenib, everolimus, trastuzumab, lapatinib, and rituximab.

11. The method for predicting long-term efficacy of a VEGF inhibitor according to Claim 9 or 10, wherein
the antitumor therapy is combined therapy composed of medication therapy using the molecular-targeted drug and medication therapy using the chemotherapy agent.

12. The method for predicting long-term efficacy of a VEGF inhibitor according to any one of Claims 1 to 11, wherein
the tumor is a recurrent tumor.

13. The method for predicting long-term efficacy of a VEGF inhibitor according to any one of Claims 1 to 11, wherein
the tumor is a metastatic focus.

14. The method for predicting long-term efficacy of a VEGF inhibitor according to any one of Claims 1 to 11, wherein
the tumor is a primary focus.

15. The method for predicting long-term efficacy of a VEGF inhibitor according to any one of Claims 1 to 14, wherein
the tumor is one or more tumors selected from the group consisting of colorectal cancer, colon cancer, rectal cancer, lung cancer, liver cancer, breast cancer, ovarian cancer, prostatic cancer, renal cancer, esophageal cancer, head and neck cancer, uterine cancer, and cervical cancer.

16. The method for predicting long-term efficacy of a VEGF inhibitor according to any one of Claims 1 to 15, wherein
the tumor is present in a plurality of places in the body of the subject.

17. The method for predicting long-term efficacy of a VEGF inhibitor according to any one of Claims 1 to 16, wherein
the mutant is one or more mutations selected from the group consisting of G12A, G12C, G12D, G12R, G12S, G12V, G13D, G12S2, G13A, G13S, G13V, G13R, G13C, Q61 H, Q61 L, Q61 R, A146T, and A 146V of the RAS protein.

18. The method for predicting long-term efficacy of a VEGF inhibitor according to any one of Claims 1 to 17, wherein
by investigating whether or not the wild-type RAS gene-derived nucleic acid is detected from circulating DNA in the blood sample and investigating whether or not the mutant RAS gene-derived nucleic acid is detected from circulating DNA in the blood sample, whether or not the RAS gene-derived nucleic acid is present in the blood sample and whether the RAS gene-derived nucleic acid is a wild type or a mutant are determined.

19. The method for predicting long-term efficacy of a VEGF inhibitor according to any one of Claims 1 to 18, wherein
the blood sample is peripheral blood, serum, or plasma.
